# EUROPEAN PATENT APPLICATION

(11) **EP 3 748 647 A1**
(43) Date of publication of application: **09.12.2020**
(21) Application number: 19305730.4
(22) Date of filing: 05.06.2019
(51) Int. Cl.: G16H 50/20

(54) **METHOD FOR DETECTING RISK OF TORSADES DE POINTES**

(71) Applicant: Assistance Publique Hôpitaux de Paris, 75004 Paris (FR)
(72) Inventor: The designation of the inventor has not yet been filed
(74) Representative: Flesselles, Bruno F.G.

(57) **Abstract**

The invention relates to methods and devices for the detection and prediction of the risk for a patient to have a torsade de pointe event, and causes thereof, in particular via the use of neural networks.

## Description

The invention relates to the detection of the risk for a patient for having a torsade de pointes event, and the mechanisms underlying such risk, in particular via the use of neural networks.

Torsades de Pointes (TdP) is an arrhythmia that can deteriorate to ventricular fibrillation and sudden death. It occurs essentially in the congenital long QT syndromes or as a rare side effect of QT-prolonging cardiac and non-cardiac drugs.

The current detection strategy is based on the QT measurements (i.e. interval between the beginning of the QRS complex and the end of the T wave, traditionally, using the lead II of the electrocardiogram (ECG)). TdP risk is a major issue for the drug industry, and can be the reason for withdrawing drugs from market.

Another method was described in US 20190059764 which discloses an algorithm using the QT interval corrected with respect to the heart rate, the maximum amplitude of the first peak of the T wave and the interval between the maximum first peak of the T wave and the end of the T wave. Although efficient, this algorithm may not always be easy to implement, as it requires comparing the ECG before anf after intake of a QT prolonging drug.

It is important to find novel ways to quantify the risk of developping TdP from drug intake. Indeed, looking at the QT only is not sufficient to detect intake of a drug which prolongs QT (sotalol). Sotalol is a potassium-current inhibitor drug, mainly used to prevent atrial fibrillation, angina pectoris and hypertension. Sotalol can lead to a prolongation of the ventricular repolarization and, rarely (1.8-4.8%), to TdP.

Recent advances in the fields of machine learning (ML) and more specifically Deep Learning (DL) have demonstrated ability to process with bio-signals including electrocardiograms. Specifically, deep learning has been used for several applications using ECG signals. In particular, convolutional neural networks (CNN) architectures were applied to detect various type of arrhythmia (Acharya, Oh, et al. 2017), myocardial infarction ((Acharya, Fujita, Lih, Hagiwara, et al. 2017); (Acharya, Fujita, Oh, et al. 2017); Liu et al. 2018), coronary artery disease and shockable ventricular arrhythmia. Most of these studies used a single-lead approach and no clinical data were included in the models.

Another recent study trained a model on single lead ECG end-to-end to detect up to 12 different arrhythmias. The database included recordings from more than 29000 subjects annotated by certified cardiographic technicians. The model tested with 336 records from 328 patients outperformed the average score of a group of 6 board-certified cardiologists (Rajpurkar et al. 2017). The same team repeated the analysis with a dataset of more than 53.000 subjects. The model tested on an independent dataset obtained an area under the curve of 97%, whereas the average cardiologist got 78% (Hannun et al. 2019). End-to-end models were also used by Pourbabaee, Roshtkhari, and Khorasani 2017, who studied paroxysmal atrial fibrillation.

Other studies focused on models with multi-lead ECGs input. For the detection of generalized anterior myocardial infarction, Liu et al. 2018 used a 4-lead approach that led to accuracies >90% with a 5-fold cross-validation. Tison et al. 2018 created instead a Hidden Markov Models-CNN model that took as an input 12 leads in order to detect pulmonary arterial hypertension, hypertrophic cardiomyopathy, cardiac amyloid and mitral valve prolapse. The area under the ROC curve was in the 77%-94% range for the 4 classes. 12 leads were also used by Attia et al. 2019, who applied a CNN model on a database of >97.000 patients to detect left ventricular dysfunction. They used a holdout dataset of >52.000 patients and an overall accuracy of 86%. Moreover, a subset of the patients that were erroneously classified as ventricular dysfunction, developed in the years to come a low ejection fraction, suggesting that the model was able to detect this condition before it becomes visible.

It is important to find novel ways to quantify the risk of developping TdP from drug intake. Indeed, looking at the QT only is not sufficient to detect intake of a drug which prolongs QT, such as sotalol, as shown in the examples.

Due to the rarity of the TdP event, and to the fact that this event occurs essentially after intake of QT-prolonging drug, the strategy developed by the inventors was to develop a model that is able to detect the intake of sotalol (which is a marker of the risk to have a torsade de pointes). Using the model so developed, the inventors were able to show that it is able to discriminate between congenital long QT syndromes (an inherited disorder of cardiac repolarization that predisposes to syncope and to sudden death from polymorphic ventricular tachycardia, i.e TdP), and in particular to identify LQT2 patients vs LQT1 or LQT3 patients (for which the biological mechanisms at hand are different).

It is to be noted that the risk mentioned above is not an absolute risk, but rather a relative risk. The patient detected "at risk" in the context of the present invention presents a higher risk than the population as a whole.

This is because the methods and systems herein disclosed are able to classify the patient in classes (increased risk / no increased risk) and in subclasses pertaining to the type of risk (or mechanism by which the risk is present) for patients in the increased risk class (drug_induced risk, acquired risk, congenital long QT syndrome and type thereof).

The invention relates to a method for producing a machine learning algorithm (or machine learning system) capable of predicting or estimating the risk for a patient to have a torsade de pointes event, and preferably the cause (or underlying mechanism) thereof, comprising:
a. storing in an electronic database patient data comprising a first parameter that is ECG from the patient, a second parameter relating to the risk for the patient to have a torsade de pointe event, and a third parameter relating to the cause thereof;
b. providing a machine learning system; and
c. training the machine learning system using the patient data, such that the machine learning system is trained to produce a prediction on the risk for the patient to have a torsade de pointe event and cause thereof when exposed to an ECG from a patient.

As demonstrated in the examples, the inventors have shown that using electrocardiograms from patients make it able to classify the patients as having a risk for torsade de pointes (as correlated with the intake of sotalol), and is able to classify the mechanism underlying the risk (intake of a drug, congenital long QT syndrome, type of congenital long QT syndrome). The fact that it is possible to obtain a system that makes it possible to detect such risk (and the mechanism underlying the risk), using the data set as disclosed above was surprising for the inventors. Indeed, the inventors show that the ECG signal contains information that can be extracted from the machine learning system and allows a very detailed classification of the patients for the TdP risk.

As such, the output of the system that is trained may rely on a classification technique which provides a prediction, probability or likelihood, rather than certainty, that the patient has a certain condition (here to risk a TdP event, and the nature of the biological mechanism). For instance, classifiers using one or more of the following techniques may be used to determine the likelihood of such risk: linear discriminant analysis, neural network, clustering techniques, support vector machine, logistic regression, naive Bayes, random forest, decision tree, etc.

It is however preferred when the machine learning classifier is an artifical neural network, and preferably a convolutional neural network.

Thus, and more specifically, the invention relates to a method for producing an artificial neural network system capable of predicting or estimating the risk for a patient to have a torsade de pointes event, and preferably the cause (or underlying mechanism) thereof, comprising:
a. storing in an electronic database patient data comprising a first parameter that is ECG from the patient, a second parameter relating to the risk for the patient to have a torsade de pointe event, and a third parameter relating to the cause thereof;
b. providing a network of nodes interconnected to form an artificial neural network, the nodes comprising a plurality of artificial neurons, each artificial neuron having at least one input with an associated weight; and
c. training the artificial neural network using the patient data such that the associated weight of the at least one input of each artificial neuron of the plurality of artificial neurons is adjusted in response to respective first, second and third parameters of a plurality of different sets of data from the patient data, such that the artificial neural network is trained to produce a prediction on the risk for the patient to have a torsade de pointe event and cause thereof when exposed to an ECG from a patient.

Using such input data and training the machine learning system, preferably the neural network, allows to obtain models that are able to determine whether an ECG of a patient presents the same characteristics than the ECG of patients at risk of having a TdP event. Such models can thus be used for implementing various methods as disclosed below.

The invention thus relates to a computer-implemented electrocardiogram (ECG) analysis method comprising:
a. isolating a data segment from an ECG signal acquired from a subject and extracting such data segment
b. optionally normalizing or standardizing the data segment
c. applying the data segment to a machine-learning model configured to detect variations in the ECG data indicative of a risk for torsade de pointes,
d. obtaining an output from the machine learning model,
wherein the output provides an estimate of the risk of having a torsade de pointes event and underlying mechanism of such risk.

This method, as well as other methods herein disclosed is performed *ex vivo.*

In particular, the machine-learning model (or system) is a neural network, such as a feedforward neural network, a convolutional neural network, or a recurrent neural network. It is preferably a convolutional neural network.

Step a. corresponds to the extraction of a segment (part) from an ECG signal (preferably during a 10 seconds window) in order to obtain a data segment that can be used for processing in a machine learning system that has previously been trained as disclosed above. It can be done online or offline.

In step b., it is possible to normalize and/or to standardize the data segment or no processing at all. For instance, when the ECG signal has been acquired on multiple leads, the standardization can be performed using the mean of the signals acquired for each lead for the patient or per each lead. Normalization may be performed using the ECG signals of all patients that are present in a reference database or for each ECG.

In step c., the data segment is applied to the machine learning algorithm that has been developed as disclosed above, in order to obtain an output. Other data can be also applied together with the data segment (such as sex, age of the patient, kaliemia...).

The invention also relates to a method for estimating the risk for a patient to have a torsade de pointes event and underlying mechanism thereof, comprising:
a. receiving, by a processing device, signal data representing a time segment of an ECG waveform of a subject patient
b. analyzing, by the processing device via a configured artificial machine learning algorithm (in particular a neural network), said signal data to generate an output of the artificial neural network,
wherein the output is the likelihood of the risk for the patient to have a tosade de pointes event, and the cause (underlying mechanism) thereof.

For the methods disclosed above (and throughout the present disclosure), the output provides a likelihood of the patient belonging to the classes that were defined during training.

As illustrations
- if the training of a neural network is performed with two classes (having ingested sotalol or not): the output will provide a likelihood of being in one or the other class
- if the training of the neural network is performed with more than two classes (for example 5 classes: sotalol+, control, long QT type 1, long QT type 2, loing QT type 3), the output will provide a likelihood for the patient of being in each of the five classes. It is clear that the output will likely favor one class indicating that the patient is probably belonging to this class.

The risk of having a torsade de pointes event is linked to the belonging, for the patient, to a class in which patients are more at risk. In other words, the risk is a relative risk as compared to patients classified as control. The risk of having a TdP event is considered to be increased (as compared to control patients) when the ECG of the patient is classified like an ECG of a patient belonging to a class of patient with increased risk (patient taking a drug that increases the QT, or patient with a long QT syndrome). The machine learning algorithm is also able to discriminate the class and thus to provide information as to the underlying mechanism that increases the TdP risk: inhibition of IKr channel for patients taking sotalol or LQT-2 patients, mutation in the KvLQT1 gene (also known as KCNQ1) involved in the IKs channel for LQT-1 patients, mutation in SCN5A, the gene encoding the sodium channel INa/INa late for LQT-3 patients.

It is thus possible to act adequately for the physician (stop medication and check that ECG becomes normal again, request genetic confirmation for long QT syndrome patients and prescribe adequate medication).

The invention also relates to a cardiac monitoring device comprising:
a. at least one electrode for obtaining an electrocardiogram (ECG) signal from a patient;
b. a processing unit comprising at least one processor operatively coupled to the at least one electrode; and
c. at least one non-transitory computer-readable medium comprising program instructions that, when executed by the at least one processor, causes the cardiac monitoring device to:
   i. obtain the ECG signal from the at least one electrode;
   ii. optionally standardize/normalize the ECG signal to obtain a standardized/normalized ECG signal;
   iii. optionally extract data from the standardized ECG signal corresponding to a predeterminated duration of time in order to obtain an extracted standardized ECG signal,
   iv. provide the ECG signal, optionally standardized/normalized and extracted, to a machine learning classifier,
thereby obtaining an output from the machine learning classifier, said output being the likelihood of having a TdP event, and cause thereof.

The standardization / normalization of the ECG signal in ii. is optional and can be performed as disclosed above. Step iii. Aims at using only the signal for a specific duration (generally 10 seconds) in the machine learning classifier. This step is optional and is not always necessary.

As indicated above, the machine learning classifier is preferably a neural network, in particular a convolutional neural network.

The invention also pertains to a system for the evaluation of the risk for a patient to have a torsade de pointe event, comprising:
a. an external defibrillator, configured to monitor electrocardiogram (ECG) data from the patient;
b. one or more processors configured to perform operations comprising:
   i. optionally performing a mathematical standardization of the ECG data for a given time domain,
   ii. performing a calculation by a machine learning classifier
   iii. and determining a likelihood for the patient to have a torsade de pointe event and cause thereof.

It is reminded that, when multiple leads are used, the ECG data is the vector formed by all these multiple ECG signals from the different leads. The examples provide an illustration of such vector for 8 leads.

In the above embodiments, the defibrillator or monitor comprising the at least one electrode and the computer/processor or processing unit can be at different locations. In this embodiment, the ECG signal is sent to the processing unit or processor in order for the machine learning classifier to process it. it is also possible to send the output to the defibrillator or monitor or to another device (such as a smartphone). In this case, it is possible to perform a monitoring at home of the risk of having a torsade de pointe event, or its evolution (depending on the evolution of the likelihood calculated by the machine learning classifier). When the defibrillator or device having the at least electrode and the processing system are distant, it is preferred when communications between these systems are encrypted.

The invention also makes it possible to evaluate the torsadogenic potential of a drug, of a product or of a composition of interest.

It is indeed important to be able to evaluate the torsadogenic potential of a compound, during clinical trials prior to the obtaining of a marketing authorization, in order to be able to draft the possible precautions for use of this drug. The torsadogenic potential of a substance is the potential of this substance to induce a torsade de pointes after administration in a patient.

The FDA (*Food and Drug Administration*) and the EMA (*European Medicines Agency*) have published guidelines resulting from the International Conference for Harmonization (ICH) on how to carry out these trials, under the title *E14 Clinical Evaluation of QT*/*QTc Interval Prolongation and Proarrhythmic Potential for Non-Antiarrhythmic Drugs.* These guidelines are accessible on the website of this agency, and are accompanied by questions and answers.

The address of the guidelines is: http://www.fda.gov/downloads/drugs/guidancecomplianceregulatoryinformation /guidances/ucm073153.pdf

As discussed above, the QT interval is an imperfect biomarker for the risk of proarrhythmic effect. In particular, the examples show that the AUC obtained when using QT for detecting patient having had sotalol is 0.76, which proves that improvement can be obtained

It is therefore required to rigorously characterize the effects of new drugs with regard to the risk of delaying cardiac repolarization. The machine learning classifier defined above makes it possible to detect whether such risk is present, by analyzing the ECG of patients having been administered the compound of interest.

The invention thus relates to an *ex vivo* method for determining the risk for a substance to induce a torsadogenic effect after administration to a patient, comprising the following steps:
a. Obtaining ECG data from the patient after administration of said composition;
b. applying the ECG data to a machine learning classifier (as disclosed above) configured to detect variations in the ECG data indicative of increased risk of torsade de pointes event,
c. obtaining an output from the machine learning classifier, wherein the output provides a likelihood of the risk for the patient to have a torsade de pointe event,
wherein the substance presents a risk of induction of a torsadogenic effect if a risk for the patient to have a torsade de pointe event is obtained after administration of the substance.

It is preferred when multiple ECG signals, acquired at various time points after administration of the substance of interest, are applied to the machine learning classifier.

It is also possible to apply, to the machine learning classifier, ECG signals for the patient, acquired before administration of the substance of interest.

Generally, and in order to limit patient variability, the method disclosed above is repeated on a cohort of patients containing a number of patients greater than or equal to 2. Carrying out these studies on a sufficient number of patients thus makes it possible to obtain results which present a statistical reality for the molecule that it is desired to test (drug of interest), eliminating the inter-patient variations.

A placebo (negative control), a positive control (such as sotalol at 80mg) and the substance of interest can be compared.

Sotalol at 80 mg is indeed preferably used as positive control. This molecule, at this concentration, has many advantages:
- the maximum concentration is well known (approximately three hours after oral administration), which makes it possible to easily plan the moment at which to take the measurements measured above.
- This molecule is known to inhibit the IKr channel.
- This molecule, at this concentration, does not induce an episode of torsade de pointes, and is therefore safe for the patient. It is however known that this molecule, at its clinical used doses (used up to 320 mg), can induce torsades de pointes.
- The machine learning classifiers herein disclosed are particularly adapted to detect intake of sotalol (80 mg) in patient with an AUC of 0.99 (CI [0.98;1]).

When testing the substance on females, it is advisable to carry out the various measurements at the different time of the menstrual cycle. This is because it is known that the level of hormones influences the risk, in a woman, of repolarization disorders (long QT).

In general, as indicated above, the above steps are carried out for each patient of a cohort of patients, and the risk is thus obtained for each patient, and for each substance.

It is preferable for the cohort of patients (the number of patients on which the substance of interest is tested) to be at least 10 patients, preferably at least 20 or even 50 or 100 patients. Those skilled in the art will be capable of determining the minimum number of subjects in the cohort of patients in order to obtain results which are statistically significant.

Likewise, before carrying out these studies, it is preferable for those skilled in the art to have carried out pharmacokinetic studies and dose-response studies on the substance of interest, in order to determine the best times to perform the ECG acquisition and machine learning classification.

The invention also relates to a method for classifying (determining) the nature of a congenital Long QT syndrome in a patient, comprising the steps of
a. Obtaining ECG data from the patient;
b. applying the ECG data to a machine learning classifier configured to detect variations in the ECG data indicative of the risk of torsade de pointes event,
c. obtaining an output from the machine learning classifier, wherein the output provides a likelihood of the nature of the long QT and classification thereof.

In particular, this method can be performed on a patient, which has been detected as having a congenital long QT syndrome, but for which the genetic analysis has not yet been performed.

This method thus makes it possible to adapt a treatment very early before the genetic confirmation is obtained (as it can take a few months).

The invention also relates to a method for detecting (or estimating) the risk for a given patient to present an episode of torsade de pointes, comprising the steps of:
a. Obtaining ECG data from the patient;
b. applying the ECG data to a machine learning classifier configured to detect variations in the ECG data indicative of a risk for the patient to have a torsade de pointe event,
c. obtaining an output from the neural network system, wherein the output provides a likelihood of the risk for the patient to have a torsade de pointe event.

It is also possible to classify or assign the patient in a class (nature of the long QT syndrome, increased risk of TdP event) by using a voting system as disclosed below. As shown in the examples, such system will increase the accuracy of the methods.

In a preferred embodiment, the likelihoods (probabilities or estimates) for a patient are calculated by averaging risk scores obtained when multiple ECG signals for the patient are applied to the machine learning classifier (voting system). The repetition of the machine learning classifier on multiple ECG samples is equivalent to a physician looking at different ECG samples to reach a conclusion. In fact, this method makes it possible to classify the patient in a given class, which is the one that is the most probable in view of the different likelihoods an risks calculated by the machine learning classifier. It is possible to process multiple ECG from the same patient were processed by the models and to affect the patient in a class when the majority of the outputs indicate a higher probability of the patient of being in that class.

It is also possible to classify the patient in a given class by using different machine classifiers that have been independently trained for the same purpose. Using this kind of voting system is equivalent of multiple physicians looking at the same data before reaching a decision.

It is also possible to combine these two voting approaches.

It is reminded that the machine learning system makes it possible to provide a probability of the patient being in the classes that have been used during the training process. During the operational phases, it is important to be able to assign a class to a patient (or a patient to a class).

The methods above can be completed by a method, wherein the patient is assigned to a class by
a. repeating the methods disclosed above with different ECG signals from the same patient and
b. assigning the patient in the class for which the majority of the outputs indicate the highest probability.

It is also possible to use a method, wherein the patient is assigned to a class by
a. repeating the methods with ECG signals from the patient using from different machine learning classifiers trained and obtained according to the methods disclosed above and
b. assigning the patient in the class for which the majority of the outputs indicate the highest probability.

The ECG signal can be acquired on a single lead (preferably the V3 lead). It is however preferred when the ECG signal is acquired from more than one lead, preferably from at least 3, more preferably from at least 6 or from at least 8 leads.

### DESCRIPTION OF THE FIGURES

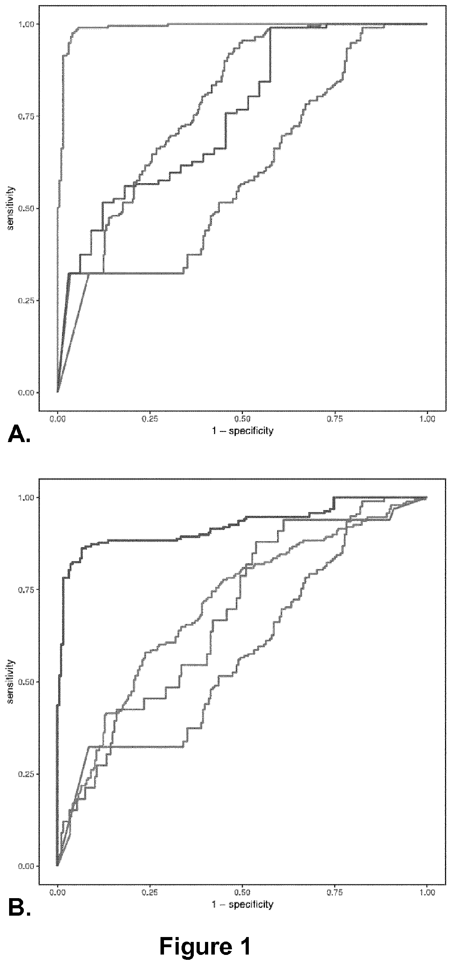

Figure 1: A. ROC curves indicating the separation between Sot+ subjects and (from left to right) controls, LQT-1, LQT-3 and LQT-2 subjects. B. ROC curves indicating the separation between LQT-2 subjects and and (from left to right) controls, LQT-1, LQT-3 and Sot+ subjects.

### EXAMPLES

### Experimental design

The training database included 9014 ECG signals from 792 healthy patients, before (n= 4014; Sot-) and after (n= 5000) Sotalol intake (80 mg per os, Sot+). The ECG were taken at different timpe points (1, 2, 3 and 5 hours after sotalol intake).

A subset of the dataset was isolated to test the trained models composed of 198 healthy patients with ECGs before (n=999) and after (n=1238) sotalol intake.

Moreover, a third dataset composed of long congenital QT patients was used to test the trained models, specifically composed of LQT-1 (n=266 patients and n=560 ECGs), LQT-2 (n=188 patients and n=456 ECGs) and LQT-3 (n=33 patients and n=67 ECGs).

### Cohort description

Two cohorts of patients were used.

The first cohort contained electrocardiograms from 990 healthy subjects before and after the consumption of 80mg of Sotalol at different periods of time (namely at 2, 3, 4 and 5 hours after Sotalol intake). Each patient had a different number of recordings, as these recordings were either repeated or not at each time interval. Each subject had at least one recording at basal and T3. Sotalol concentration was measured in serum, at 2, 3 and 5 hours after intake, but this not for all subjects. Before the Sotalol intake, all signals were associated to a zero concentration of Sotalol. This first cohort was used to train the CNN (Convolutional Neural Network) and ResNet models that classify individuals in Sot+/Sot- groups and predict the concentration of Sotalol in the blood.

A second cohort composed of long-QT congenital patients was also used, specifically composed of LQT-1 (n=266 patients and n=560 ECGs), LQT-2 (n=188 patients and n=456 ECGs) and LQT-3 (n=33 patients and n=67 ECGs), from a second study to test the trained models.

### Convolutional Neural Network architecture

Two types of models were developed, depending on the inputs. Either just the ECGs (M1) or the ECGs with the data associated to the sex, age and Kalemia (M2) were used. The models' architecture is the same

The models were based on convolutional neural networks (CNN), composed of 11 blocks of convolution and each block had two Conv1D layers with the same number of filters. The number of filters for each block were 8, 8, 8, 16, 32, 64, 128, 256, 512, 1024 and 2048 respectively (11 blocks in total). Zero padding was used for both Conv1D in order to have an output with the same length of the input (option "same"). Adam optimizer, binary cross-entropy loss and early stopping (patience=50) were used. The class weights were computed to balance the output classes before training. The remaining model parameters had default values. In the M2 model, the clinical data were concatenated to the flattened layer output, before the fully connected neural network.

Different deep learning models based on the same CNN architecture were developed. The first (M1), included only ECG data before and after Sotalol intake (respectively denoted below as Control and Sot+), while the other (M2) included also basic clinical information, namely, age, sex and kalemia quantified at study baseline.

Different models M1 were developed. They all presented the same whole performance, although with some differences in assessment for some individual during the testing phase.

### Preparation of the ECG data

Raw ECG was used, acquired with the Cardioplug device (Cardionics Inc). The ECGs were associated to 8 leads (I, II, V1, V2, V3, V4, V5, V6). Each recording lasted 10s, with a sample size of 500 Hz. The data in the format of .SCP files were parsed using the Biosig library (http://biosig.sourceforge.net/index.html).

Similarly, raw ECG data from the long-QT patient was used, acquired with the CalECG tool from 1992 to 2018. Raw .xml files were parsed using the python xmltodict library. In total, 875 files were acquired at 500 Hz, and 208 were acquired at 250 Hz, for a total of 1083 recordings. The 250 Hz signals were up-sampled to 500 Hz using a cubic interpolation (interp1d function from the scipy python library).

No filtering, nor any other transformation was applied to the data considered in these studies, except for standardization which was performed at the whole ECG level (i.e. each lead signal is standardized by the mean of all other lead signals for a given ECG). The data were stored in python dictionaries that were used for training the models.

The ECG data used corresponds to a segment of 10 consecutive seconds of a greater ECG acquisition.

For training, the data was entered as a 3 D tensor (8 leads, 5000 time points for each lead, Sot+ or Sot-). For testing the models, the data was entered as a 2D tensor (8 leads, 5000 time points for each lead).

### Model training and evaluation

All the models explored were created using the Keras library in Python. In both classification and regression experiments, the first database was split in Training (80%) and Holdout (20%) sets. 10-times 10-fold cross-validation were performed in the Training database to estimate the generalization confidence of the algorithms. At each fold, the train subset and the test subset had distinct examples and both sets (i.e. training and testing) were normalized using the average and standard deviation of the CV training set to avoid overfitting. The final models (M1, M2 and ResNet18) were trained in the whole training set and were tested in the holdout and the external long-qt congenital cohort. Estimated CV empirical and generalization performance is also provided.

For the classification task, the training dataset included 9014 ECG signals from 792 healthy patients, before (n= 4014) and after (n= 5000) Sotalol intake (80 mg). A subset of the dataset was isolated to test the trained models composed of 198 healthy patients with ECGs before (n=999) and after (n=1238) sotalol intake. The classification was performed as a binary classification: no drug (Sot-) vs. drug (Sot+). The recordings were used associated to inclusion, basal and T0 as Sot- and recordings after drug intake (at T1, T2, T3, T4) as Sot+. Finally, The long-qt congenital dataset were used to test the trained models, specifically composed of LQT-1 (n=266 patients and n=560 ECGs), LQT-2 (n=188 patients and n=456 ECGs) and LQT-3 (n=33 patients and n=67 ECGs).

### Voting strategy

Performances were computed using both single-signal analysis and by averaging risk scores from multiple recordings for a given patient and condition. The output provided by the models is a probability (likelihood) of being Sot+ (having ingested Sotalol).

In order to affect the patient in one of the Sot+ or Sot- classes, multiple ECG from the same patient were processed by the models and the patient was affected as being Sot+ (resp Sot-) if the majority of the outputs indicated a probability of being Sot+ (resp. Sot-) higher than 0.5 (50%).

Alternatively, a voting strategy can be used by processing one ECG from a patient with multiple models (trained separately, using similar architectures or different), and affecting the patient to the Sot+ (resp Sot-) class if the majority of the models indicate a probability of being Sot+ (resp. Sot-) higher than 0.5 (50%).

### Sotalol intake is accurately detected from raw ECG signals

The performance of the models was evaluated either at the single ECG level or averaged by patient for each of the 10-times 10-cross validation folds (CV). It was observed that the average test accuracy in CV is comparable to the holdout accuracy as is the overall empirical accuracy and the empirical in the training process (CV), indicating a good estimation of the performance of the models.

The average generalization accuracy of the model M1 is 0.885 (sd=0.003) when computed for each ECG and 0.945 (sd=0.004) when computed for each patient (using the voting strategy as disclosed above). Similarly, for the model M2, the average generalization accuracy is 0.89 (sd=0.003) when computed for each ECG and 0.947 (sd=0.003) when computed for each patient.

There is no statistical difference between M1 and M2, thus indicating that the ECG alone contains all information pertaining to the detection of the ingestion of Sotalol.

However, there was a highly significant difference between the generalization accuracy at the ECG and patient level (voting, p<1.03e-25), indicating the importance of multiple signal acquisitions.

Finally, the precision of these models is highly elevated, 0.963 (sd=0.004) and 0.961 (sd=0.003) for M1 and M2 respectively (voting) as is the recall for the same models: 0.934 (sd=0.005) and 0.937 (sd=0.004).

Altogether, these results indicate that the CNN model trained only with raw electrocardiograms is very sensitive and accurate in predicting whether a healthy subject has taken Sotalol of (80 mg) or not.

### Sotalol-intake classification models discriminate long-QT congenital profiles

Next, the simplest (M1) model trained to discriminate Sotalol-intake patients was used to classify long-QT congenital patients from the LQT-congenital cohort. The model provided a risk-probability [0,1], which was converted in a Sot-/Sot+ class based on threshold >=0.5.

First, it was noticed that most of the Sot+ ECGs (95%) are classified as Sot+ and most of the controls (87%) are classified as Sot-. The voting results display superior performance for the Sot+ (99%) and control (93%) groups as previously discussed above. Based on these observations, only the results integrating ECG signals at the patient level (voting) are later discussed.

Second, it was noticed that all LQT patients are classified in their majority as Sot+. LQT-2 display the strongest proportion (85%) of Sot+ followed by LQT-3 (67%) and LQT-1 (63%). Moreover, ROC analyses of model M1 were performed to discriminate groups together. The results for comparisons between the Sot+ vs. control, LQT-1, LQT-2 and LQT-3 groups presented AUCs (Area Under Curve) of 0.99 (Confidence Intervalle, CI [0.98;1]), 0.79 (CI [0.75;0.83]), 0.59 (CI [0.53;0.64]) and 0.75 (CI [0.66;0.84]) respectively (Figure 1.A). The results for comparisons between the LQT-2 vs. LQT-1, LQT-3, Sot+ and control groups, presented AUCs of 0.71 (CI [0.66;0.75]), 0.68 (CI [0.58;0.77]), 0.59 (CI [0.53;0.64]) and 0.92 (CI [0.89;0.95]) respectively (Figure 1.B).

As a matter of comparison, the AUC between Sot+ and controls when assessing only the QT is 0.7674 [0.7381;0.7968].

The low AUC of Sot+ vs LQT-2 indicate that this model cannot discriminate well these two groups, showing their inter-similarity (IKr channel inhibition). However, the fact that the AUC was higher than 0.5 shows that there are some differences between these two conditions and that further training (with one class for Sot+ subjects and another class for LQT-2 subjects) will allow to be able to discriminate Sot+ and LQT2 patients.

Altogether with the high AUC of LQT-2 vs. control (0.92), these results indicate that the M1 model trained with Sotalol data is relevant and pertinent as a surrogate to discriminate LQT-2 patients from controls, in case of a patient with a congenital long QT.

### Sotalol-intake classification models identifies patients with Torsade de Pointes at the time of the event

Two patients were hospitalized shortly after an episode of torsade de pointes.

ECG was performed on these patients and the ECG signals were applied to the neural network classifier as disclosed above.

For patient 1, the likelihood of being at risk of torsades de pointes (output of the model) were 0.991629, 0.989505, and 0.072291 (thre ECG).

For patient 2, the likelihood were
Visit 1: 0.991812, 0.999446and 0.984214
Visit 2 : 0.998443, 0.999096, and 0.999811.

These results illustrate an example of patients who had a TdP episode and were classified as Sot+ shortly after this episode. The patients didn't have a congenital long QT and didn't take any drug inducing QT prolongation. Their QT prolongation was acquired.

The models described above are thus able to classify patients as "at risk for Torsade de Pointes", juste after a cardiac event, which makes it possible to make a retrospective diagnosis.

### Discussion

The study above shows that that ECGs signals carry important information and are affected by the Sotalol intake, which inhibits the IKr channel.

Indeed, being able to provide an estimation of the previous intake of Sotalol (or of another drug) in the blood may lead to a better understanding of the risk of TdP.

QT prolongation has been shown to be associated to TdP and therefore many studies considered it as a TdP surrogate. However, recent studies demonstrated that QT prolongation alone is not a reliable predictor due to the high false positive and false negatives rates (Hondeghem, 2018; see also AUC for discrimintating Sot+ and contrils using QT alone).

The models developed herein were able to accurately classify, with an overall generalization accuracy >94%, if the patient did or did not take Sotalol, regardless of the time after drug consumption (within 4 hours). It was shown that multiple acquisitions taken together with a voting approach improved the classification. *Per* se, this demonstrates presence of a rich information contained within an electrocardiogram, richer than QT only.

Next, it was shown that this surrogate model could also discriminate long-qt congenital patients at different degrees based on the type of mutation LQT-1, LQT-2 and LQT-3. This is very important as these mutations are rare and difficult to lean.

Such classification method is very useful in the case of compliance control and to adjust the dose in the context of drug monitoring. Moreover, given the importance of lowering healthcare related costs, this system would be cheaper, more practical and faster than standard blood analysis. It would allow to also have a continuous drug-estimation instead of discrete results.

Attia et al., 2018 tried to estimate plasma drug concentration using CNN by analyzing 10-s recordings of 42 patients that received Dofetilide (antiarrhythmic drug) or placebo. These recordings were recorded at specific timepoints before and after drug consumption. In this experiment the authors used the data from two distinct prospective randomized controlled trials available in the Physionet repository. In their regression analysis, using CNN, they reached a correlation of 0.85. However, the database was relatively small (42 subjects) and no cross-validation in training was used, leading to possible overfitting.

Yιldιrιm et al. 2018 were able to detect 17 types of arrhythmias with an accuracy of 91% (test set) using an end-to-end model. However, this analysis was based on 1000 single-lead ECGs of 10 seconds that belonged to 45 people. This means that the same subject had data both in training and testing, leading to non-independent datasets and overfitted models. Other authors applied data-augmentation in order to increase the dataset to detect 5 heart conditions and accuracy was above 90% as tested with 10-fold cross-validation procedure (Acharya, Oh, et al. 2017). These authors also compared the performances between a denoised and raw database, showing that noise removal was not associated to a higher score. However, the cross-validation random split was done after the data augmentation. The fact that the signals were not completely independent between the train and test set might lead to overfitted performances.

### Other developments

Various models were also developed:
When using only one lead, it was shown that the same type of results (accuracy) could be obtained than for the models described in details above that use the ECG from 8-leads.

Another model is developed, using as the training set, ECG from controls, patients having ingested sotalol, LQT-1, LQT-2 and LQT-3 patients. This model will provide, as the output the probability for the patient to be in each of the five classes. Using a voting system (preferably by providing multiple ECG of the patient or alternatively by using multiple CNN models), it is possible to provide information for the physician and allows an appropriate care

Models can also be developed and enriched, using multiple TdP potentially inducing drugs to be able to discriminate the drug that has been taken by the patient.

### REFERENCES

Acharya, U. R., Fujita, H., Lih, O. S., Hagiwara, Y., Tan, J. H., & Adam, M. (2017). Automated detection of arrhythmias using different intervals of tachycardia ECG segments with convolutional neural network. Information sciences, 405, 81-90.
Acharya, U. R., Fujita, H., Oh, S. L., Hagiwara, Y., Tan, J. H., & Adam, M. (2017). Application of deep convolutional neural network for automated detection of myocardial infarction using ECG signals. Information Sciences, 415, 190-198.
Acharya, U. R., Oh, S. L., Hagiwara, Y., Tan, J. H., Adam, M., Gertych, A., & San Tan, R. (2017). A deep convolutional neural network model to classify heartbeats. Computers in biology and medicine, 89, 389-396.
Attia et al. 2019, J Cardiovasc Electrophysiol. 2019 Feb 28
Attia, Z. I., Sugrue, A., Asirvatham, S. J., Ackerman, M. J., Kapa, S., Friedman, P. A., & Noseworthy, P. A. (2018). Noninvasive assessment of dofetilide plasma concentration using a deep learning (neural network) analysis of the surface electrocardiogram: A proof of concept study. PloS one, 13(8), e0201059.
Hannun et al. 2019, Nat Med. 2019 Jan;25(1):65-69
Hondeghem, L. M. (2018). Drug-Induced QT Prolongation and Torsades de Pointes: An All-Exclusive Relationship or Time for an Amicable Separation?. Drug safety, 41(1), 11-17.
Liu et al. 2018, IEEE J Biomed Health Inform. 2018 Sep;22(5):1434-1444 Pourbabaee, Roshtkhari, and Khorasani 2017 IEEE Transactions on Systems, Man, and Cybernetics: Systems PP(99):1-10 . June 2017
Rajpurkar, P., Hannun, A. Y., Haghpanahi, M., Bourn, C., & Ng, A. Y. (2017). Cardiologist-level arrhythmia detection with convolutional neural networks. arXiv preprint arXiv:1707.01836.
Tison et al. 2018, Int J Med Inform. 2018 Dec;120:1-7
Yιldιrιm et al. 2018, Comput Biol Med. 2018 Nov 1;102:411-420

## Claims

1. A computer-implemented electrocardiogram (ECG) analysis method comprising:
a. isolating a data segment from an ECG signal acquired from a subject and extracting such data segment
b. optionally normalizing or standardizing the data segment
c. applying the data segment to a machine-learning classifier configured to detect variations in the ECG data indicative of a risk for torsade de pointes,
d. obtaining an output from the machine-learning classifier,
wherein the output provides an estimate of the risk of having a torsade de pointes event and underlying mechanism thereof.

2. A method for estimating the risk for a patient to have a torsade de pointes event and mechanism thereof, comprising:
a. receiving, by a processing device, signal data representing a time segment of an ECG waveform of a subject patient
b. analyzing, by the processing device *via* a configured artificial machine learning classifier, said signal data to generate likelihoods as output of the artificial neural network,
wherein the likelihoods relate to the risk for the patient to have a tosade de pointes event, and the underlying mechanism thereof.

3. A method for producing a machine learning classifier capable of estimating the risk for a patient to have a torsade de pointes event, and underlying mechanism thereof, comprising:
a. storing in an electronic database patient data comprising a first parameter that is ECG from the patient, a second parameter relating to the risk for the patient to have a torsade de pointe event, and a third parameter relating to the cause thereof;
b. providing a machine learning system; and
c. training the machine learning system using the patient data, such that the machine learning system is trained to produce a prediction on the risk for the patient to have a torsade de pointe event and cause thereof when exposed to an ECG from a patient.

4. A method for producing an artificial neural network capable of estimating the risk for a patient to have a torsade de pointes event, and cause thereof, comprising:
a. storing in an electronic database patient data comprising a first parameter that is ECG from the patient, a second parameter relating to the risk for the patient to have a torsade de pointe event, and a third parameter relating to the cause thereof;
b. providing a network of nodes interconnected to form an artificial neural network, the nodes comprising a plurality of artificial neurons, each artificial neuron having at least one input with an associated weight; and
c. training the artificial neural network using the patient data such that the associated weight of the at least one input of each artificial neuron of the plurality of artificial neurons is adjusted in response to respective first, second and third parameters of a plurality of different sets of data from the patient data, such that the artificial neural network is trained to produce a prediction on the risk for the patient to have a torsade de pointe event and cause thereof when exposed to an ECG from a patient.

5. An *ex vivo* method for determining the risk for a substance to induce a torsadogenic effect after administration to a patient, comprising the following steps:
a. Obtaining ECG data from the patient after administration of said composition;
b. applying the ECG data to a machine learning classifier configured to detect variations in the ECG data indicative of increased risk of torsade de pointes event,
c. obtaining an output from the machine learning classifier, wherein the output provides a likelihood of the risk for the patient to have a torsade de pointe event,
wherein the substance presents a risk of induction of a torsadogenic effect if a risk for the patient to have a torsade de pointe event is obtained after administration of the substance.

6. The method of claim 5, which is repeated on a cohort of patients containing a number of patients greater than or equal to 10.

7. A method for determining the nature of a congenital Long QT syndrome in a patient, comprising the steps of
a. Obtaining ECG data from the patient;
b. applying the ECG data to a machine learning classifier configured to detect variations in the ECG data indicative of the risk of torsade de pointes event,
c. obtaining an output from the machine learning classifier, wherein the output provides a likelihood of the nature of the long QT and classification thereof.

8. A method for detecting the risk for a given patient to present an episode of torsade de pointes, comprising the steps of:
a. Obtaining ECG data from the patient;
b. applying the ECG data to a machine learning classifier configured to detect variations in the ECG data indicative of a risk for the patient to have a torsade de pointe event,
c. obtaining an output from the neural network system, wherein the output provides a likelihood of the risk for the patient to have a torsade de pointe event.

9. The method of any one of claims 1 to 3 and 5 to 8, wherein the machine-learning classifier is a neural network, preferably a convolutional neural network.

10. The method of any one of claims 1, 2 and 5 to 9, wherein the patient is assigned to a class by
a. repeating the methods with different ECG signals from the same patient and
b. assigning the patient in the class for which the majority of the outputs indicate the highest probability.

11. The method of any one of claims 1, 2 and 5 to 9, wherein the patient is assigned to a class by
a. repeating the methods with ECG signals from the patient using from different machine learning classifiers obtained according to the method of claim 3 or 4 and
b. assigning the patient in the class for which the majority of the outputs indicate the highest probability.

12. The method of any one of claims 1 to 11, wherein the ECG signal is an ECG signal from one single lead (preferably the V3 lead).

13. The methof of any one of claims 1 to 11, wherein the ECG signal is an ECG signal from more than one lead, preferably at least 3, more preferably at least 6 or at least 8 leads.

14. A cardiac monitoring device comprising:
a. at least one electrode for obtaining an electrocardiogram (ECG) signal from a patient;
b. a processing unit comprising at least one processor operatively coupled to the at least one electrode; and
c. at least one non-transitory computer-readable medium comprising program instructions that, when executed by the at least one processor, causes the cardiac monitoring device to:
i. obtain the ECG signal from the at least one electrode;
ii. optionally standardize/normalize the ECG signal to obtain a standardized/normalized ECG signal;
iii. optionally extract data from the standardized ECG signal corresponding to a predeterminated duration of time in order to obtain an extracted standardized ECG signal,
iv. provide the ECG signal, optionally standardized/normalized and extracted, to a machine learning classifier,
thereby obtaining an output from the machine learning classifier, said output being the likelihood of having a TdP event, and cause thereof.

15. A system for the evaluation of the risk for a patient to have a torsade de pointe event, comprising:
a. an external defibrillator, configured to monitor electrocardiogram (ECG) data from the patient;
b. one or more processors configured to perform operations comprising:
i. optionally performing a mathematical standardization of the ECG data for a given time domain,
ii. performing a calculation by a machine learning classifier
iii. and determining a likelihood for the patient to have a torsade de pointe event and cause thereof.
